# EUROPÄISCHE PATENTANMELDUNG

(11) **EP 3 339 310 A1**
(43) Veröffentlichungstag der Anmeldung: **27.06.2018**
(21) Anmeldenummer: 17210473.9
(22) Anmeldetag: 22.12.2017
(51) Int. Cl.: C07D 495/04, H01B 1/12, H01L 35/00

(54) **ORGANISCHE HALBLEITER, VERFAHREN ZU IHRER HERSTELLUNG UND IHRE VERWENDUNG**

(30) Priorität: 23.12.2016 DE 102016125644
(71) Anmelder: Karlsruher Institut für Technologie, 76131 Karlsruhe (DE)
(72) Erfinder: Aslan, Silas Mehmet, 76133 Karlsruhe (DE)
(74) Vertreter: Geitz Truckenmüller Lucht Christ

(57) **Zusammenfassung**

Die Erfindung betrifft organische n-typ Halbleiter der allgemeinen Formel (I), 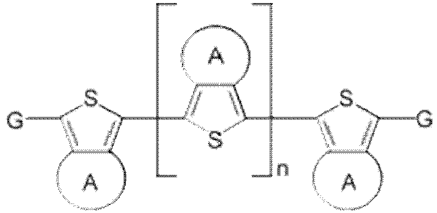 ein Verfahren zu ihrer Herstellung und ihre Verwendung.

## Beschreibung

Die Erfindung betrifft organische n-typ Halbleiter, ein Verfahren zur Herstellung der Halbleiter sowie die Verwendung der organischen n-typ Halbleiter.

Organische Halbleiter sind von großem weltweitem Interesse zur Anwendung zum Beispiel in organischen Transistoren. Leitfähige organische Materialien besitzen aufgrund ihrer nahezu unbegrenzten Ressourcen ein großes kommerzielles Potential. Eigenschaften wie ein geringes Gewicht, hohe Elastizität, Korrosionsbeständigkeit und eine sehr variable chemische Modifizierbarkeit machen sie insbesondere für den Einsatz in der Thermoelektrik interessant.

Ein thermoelektrischer Generator (TEG) ist beispielsweise aus der DE 102012105086 B4 bekannt. Dieser wandelt mit Hilfe von Thermopaaren aus organischen n-typ- und p-typ-Halbleitern eine Temperaturdifferenz unter Ausnutzung des Seebeck-Effekts direkt in eine elektrische Spannung um. Die für den jeweiligen Leiter charakteristische Thermospannung wird als Seebeck-Koeffizient bezeichnet, welcher für n-typ Leiter negativ und für p-typ Leiter positiv ist.

Organische polymere p-typ Halbleiter auf der Basis von p-dotierten Polythiophenen, wie beispielsweise Polyalkylendioxythiophenen oder Polyalkylendithiathiophenen oder Polythienothiophenen sowie deren Derivate und Verfahren zu deren Herstellung durch chemische Oxidation der entsprechenden Thiophene sind aus der einschlägigen Fachliteratur bekannt. Exemplarisch wird auf Elschner, Andreas et al.; PEDOT: Principles and Applications of an Intrinsically Conductive Polymer, ISBN 9781420069112 und L. Groenendaal, F. Jonas, D. Freitag, H. Pielartzik, J. R. Reynolds in Advanced Materials 2000, 12, Seiten 481-494 sowie der darin aufgeführten Referenzliteratur verwiesen.

Bisherige organische n-typ Halbleiter basieren meist auf n-dotiertem Naphthalendiimid (NDI) oder Perylendiimid (PDI). Die Strukturen und Substituenten dieser zweidimensional kondensierten Ringsysteme sind jedoch sehr komplex, was viele einzelne Syntheseschritte zu ihrer Herstellung erforderlich macht. Die hieraus resultierenden hohen Herstellungskosten sind dabei für einen wirtschaftlichen Einsatz der Verbindungen von großem Nachteil. Ein weiterer Nachteil ist die hohe Instabilität der mit Elektronendonoren über Elektronenübertragung n-dotierten n-typ Halbleiter. Diese reagieren sehr leicht mit Luftsauerstoff unter Neutralisation der negativen Ladungen. Eine 100%-ige Stabilität der Verbindungen ist daher nur unter Schutzgasatmosphäre gegeben und die Anwendbarkeit entsprechend beschränkt.

Vor diesem Hintergrund liegt der vorliegenden Erfindung die Aufgabe zugrunde, stabile organische n-typ Halbleiter sowie ein kostengünstiges Verfahren, mit dem diese auf einfache Weise hergestellt werden können, bereitzustellen.

Dies gelingt zum einen mit organischen Halbleitern gemäß den Merkmalen des Anspruchs 1.

Organische n-typ Halbleiter der allgemeinen Formel (I), 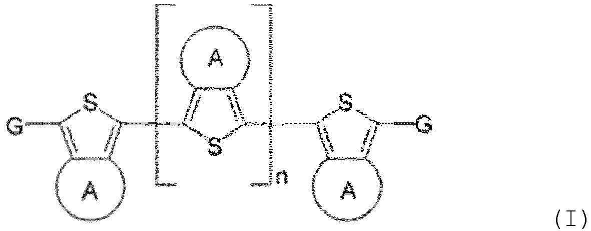 worin
A für einen gegebenenfalls ein oder mehrfach substituierten 5- oder 6-gliedrigen ungesättigten oder gesättigten heterocyclischen Ring mit bis zu zwei Heteroatomen steht, wobei die Heteroatome gleich oder verschieden sein können und ausgewählt sind aus der Gruppe aus Sauerstoffatomen und Schwefelatomen,
G ein Substituent mit einem negativen induktiven Effekt bedeutet,
und n für eine ganze Zahl von 2 bis 6 steht, bevorzugt für eine ganze Zahl von 2 bis 4 steht,
weisen einen negativen Seebeck-Koeffizienten auf. Sie eignen sich dadurch zum Einsatz als n-typ Halbleiter in elektrischen Anordnungen. Die organischen n-typ Halbleiter gemäß der allgemeinen Formel (I) zeigen darüber hinaus bereits mit einer vergleichsweise geringen Anzahl an Thiophen-Einheiten für den Einsatz als organische Halbleiter geeignete Ladungstransporteigenschaften. Zudem erweisen sich die erhaltenen organischen n-typ Halbleiter der allgemeinen Formel (I) als stabil gegenüber Luftsauerstoff.

Die allgemeine Formel (I) ist so zu lesen, dass n Thiophen-Einheiten von jeweils endständigen, mit G substituierten Thiophen-Einheiten flankiert werden. Die Substituenten G in α-Stellung zum Schwefelatom der endständigen Thiophen-Einheiten besitzen gegenüber den Thiophen-Einheiten eine elektronenziehende Wirkung und üben einen negativen induktiven (-I) Effekt aus.

Auf die Darstellung von Ladungen wurde verzichtet, da die Lokalisation der Ladungen nicht eindeutig angegeben werden kann.

Substituenten mit einem negativen induktiven Effekt sind dem Fachmann bekannt. Beispiele für bevorzugte G sind -O-Alkylgruppe, -OH, -NH-Alkylgruppe, -N-(Alkylgruppe)₂, -NH₂, -NH(CO)-Alkylgruppe, -O(CO)-Alkylgruppe, -O(CO)-Arylgruppe, -O(CO)-Phenylgruppe, Halogenatome, Halogenalkylgruppe, besonders bevorzugte G sind Fluor, Chlor, Brom oder-CH₂-CF₂-CF₃. Bevorzugt sind C₁-C₁₈-Alkylgruppen bzw. C₁-C₁₈-Halogenalkylgruppen. Die Substituenten G können gleich oder verschieden sein. Bevorzugt sind sie gleich.

In der allgemeinen Formel (I) steht A bevorzugt für 1,4-Dioxan, 1,4-Oxathian, 1,4-Dithian oder Dehydrothiophen. Bevorzugte Thiophen-Einheiten sind 3,4-Ethylendioxythiophen (2,3-Dihydrothieno[3,4-b]-1,4-Dioxin), 3,4-Ethylenoxythiathiophen (2,3-Dihydrothieno[3,4-b]-1,4-Oxathiin), 3,4-Ethylendithiathiophen (2,3-Dihydrothieno[3,4-b]-1,4-Dithiin) und Thieno[3,4-b]thiophen. Unsymmetrische Thiophen-Einheiten wie 3,4-Ethylenoxathiathiophene und Thieno[3,4-b]thiophene können Kopf-Kopf, Kopf-Schwanz, Schwanz-Kopf, Schwanz-Schwanz verknüpft sein. Weiter bevorzugt sind symmetrische Thiophen-Einheiten wie 3,4-Ethylendioxythiophene oder 3,4-Ethylendithiathiophene, da diese eine leichtere synthetische Zugänglichkeit aufweisen.

A kann gegebenenfalls durch einen oder mehrere Reste, die gleich oder verschieden sein können, substituiert sein. Der oder die Reste können an beliebigen Kohlenstoffringatomen von A gebunden sein. Die Bindung von bis zu zwei Resten an dem selben Kohlenstoffringatom ist möglich. Bevorzugte Reste sind Hydroxylreste, geradkettige oder verzweigte gegebenenfalls substituierte C₁-C₂₀-Alkylreste, gegebenenfalls substituierte C₃-C₁₂-Cycloalkylreste, gegebenenfalls substituierte C₆-C₁₄-Arylreste, gegebenenfalls substituierte C₇-C₁₈-Aralkylreste, gegebenenfalls substituierte C₁-C₆-Hydroxyalkylreste und gegebenenfalls substituierte C₂-C₂₀-Alkoxyalkylreste. Bevorzugte Alkoxyalkylreste sind solche mit einem Kohlenstoffatom im Alkyl, im Speziellen der-CH₂-O-(CH₂)₁₃-CH₃ oder der -CH₂-O-(CH₂)₁₅-CH₃ Rest.

Weitere bevorzugte Reste an A oder Substituenten an den Resten von A sind eine oder mehrere ionische Gruppen. Eine bevorzugte ionische Gruppe ist die anionische SO₃⁻Gruppe. Ebenfalls erfindungsgemäß sind Gemische der organischen n-typ Halbleiter der allgemeinen Formel (I), bei denen beispielsweise organische n-typ Halbleiter der allgemeinen Formel (I) mit unterschiedlicher Zahl für n, mit unterschiedlichen Resten an A, mit unterschiedlichen Substituenten an den Resten von A und/oder mit unterschiedlichen Substituenten G vermischt sind.

Bevorzugte organische n-typ Halbleiter der allgemeinen Formel (I) sind solche der allgemeinen Formel (I-1), 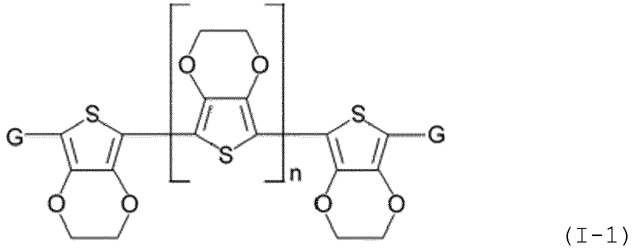 worin G und n die in der Formel (I) genannte Bedeutung haben.

Besonders bevorzugte organische n-typ Halbleiter der allgemeinen Formel (I) sind solche der allgemeinen Formel (I-1), worin G Halogenatom, insbesondere Fluor, Chlor oder Brom, oder -CH₂-CF₂-CF₃ bedeutet.

Ein weiterer Gegenstand der vorliegenden Erfindung ist ein Verfahren zur Herstellung von organischen n-typ Halbleitern der allgemeinen Formel (I). Die organischen n-typ Halbleiter der allgemeinen Formel (I), 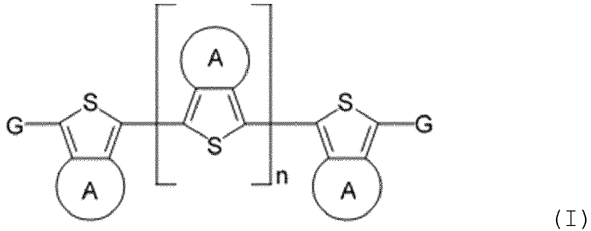 worin A, G und n die oben angegebene Bedeutung haben, können erhalten werden, wenn Thiophene der allgemeinen Formel (II), 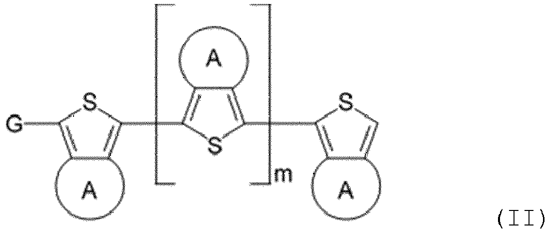 worin A und G die in der Formel (I) genannte Bedeutung haben, und m für 0, 1 oder 2 steht, bevorzugt für 0 oder 1 steht,
chemisch oxidativ umgesetzt werden.

Dies war unerwartet, da mittels chemisch oxidativer Umsetzung Thiophene, wie in der vorgenannten Literatur beschrieben, normalerweise in p-dotierte Polythiophene mit einem positiven Seebeck-Koeffizienten überführt werden. Wie bereits zu den erfindungsgemäßen organischen n-typ Halbleitern erläutert, weisen diese einen negativen Seebeck-Koeffizienten auf. Ein solcher findet sich üblicherweise bei n-dotierten Materialien.

Die Umsetzung der Thiophene der allgemeinen Formel (II) findet in einem inerten Lösungsmittel statt. Geeignete Lösungsmittel sind beispielsweise Alkohole wie Methanol, Ethanol, 2-Propanol, Butanol, Kohlenwasserstoffe wie Hexan, Heptan, Toluol, Xylol, Chlorkohlenwasserstoffe wie Methylenchlorid, Chloroform, Chlorbenzol, Ether wie Diethylether, Anisol, Sulfoxide wie Dimethylsulfoxid, Glykole wie Ethylenglykol, Diethylenglykol, Triethylenglykol, Tetraethylenglykol, Triethylenglykolmonomethylether. Bevorzugt werden Glykole, besonders bevorzugt werden Triethylenglykol, Tetraethylenglykol und Triethylenglykolmonomethylether. Es können auch Lösungsmittelgemische sowie Mischungen von Wasser mit wassermischbaren Lösungsmitteln vorteilhaft sein.

Bevorzugt werden Thiophene der allgemeinen Formel (II-1), 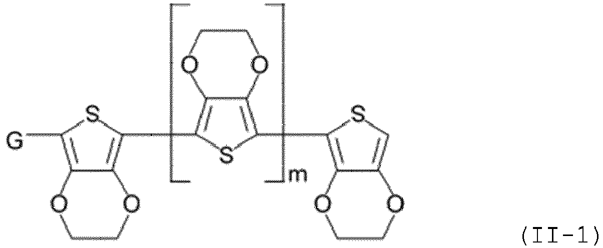 worin G ein Chloratom bedeutet und m für 0 oder 1 steht, chemisch oxidativ zu organischen n-typ Halbleitern der Formel (I-1), worin G die in (II-1) genannte Bedeutung hat und n für eine ganze Zahl zwischen 2 und 4 steht, umgesetzt.

Das Vorgehen zur Umsetzung der Thiophene erfolgt gemäß den bekannten Verfahren zur chemisch oxidativen Polymerisation von Thiophenen.

Es ist dabei für den Fachmann selbstverständlich, dass Verbindungen der allgemeinen Formel (II), bevorzugt Verbindungen der allgemeinen Formel (II-1), mit gleichen oder unterschiedlichen Zahlenwerten für m und gleichen oder unterschiedlichen Substituenten G, mit gleichen oder unterschiedlichen Resten an A und/oder Substituenten an den Resten von A miteinander zu Gemischen umgesetzt werden können.

Die Verbindungen der allgemeinen Formel (II) können gegebenenfalls in einem Gemisch mit Verbindungen der allgemeinen Formel (III), 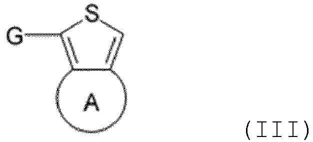 bevorzugt mit Verbindungen der allgemeinen Formel (III-1), 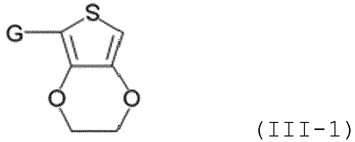 worin A und G die in der allgemeinen Formel (I) beschriebene Bedeutung haben, umgesetzt werden.

Es kann ebenfalls vorteilhaft sein, Verbindungen der allgemeinen Formel (III), bevorzugt der allgemeinen Formel (III-1), oder Mischungen von diesen umzusetzen. Bei den hieraus resultierenden n-typ Halbleitern der allgemeinen Formel (I) steht n für 0.

Als Oxidationsmittel kommen alle für die chemisch oxidative Polymerisation von Thiophenen üblichen, dem Fachmann bekannten, und beispielsweise in der eingangs genannten Fachliteratur beschriebenen Oxidationsmittel in Betracht. Geeignete Oxidationsmittel sind unter anderem anorganische Metallsalze und Metallsalze organischer Säuren von Fe³⁺, Ce⁴⁺, Cu²⁺, Au³⁺ oder Mn⁴⁺. Bevorzugte Oxidationsmittel sind Eisen-(III)-Verbindungen wie Eisen-(III)-chlorid und Eisen-(III)-tosylat. Besonders bevorzugt ist Eisen-(III)-tosylat.

Die Umsetzungstemperatur kann in Anbetracht der Vielzahl an zur Verfügung stehenden Reaktionspartnern in einem weiten Temperaturbereich variieren. Die für die jeweiligen Reaktionspartner optimale Umsetzungstemperatur ist experimentell leicht zu ermitteln. Die Umsetzung kann beispielsweise bei Raumtemperatur durchgeführt werden. Es kann jedoch auch nützlich sein, die Umsetzung bei tieferen oder höheren Temperaturen vorzunehmen, beispielsweise bei Temperaturen von -30°C bis 150°C, insbesondere von 10°C bis 130°C. Sofern der Substituent G ein Halogenatom ist oder Halogenatome enthält, wird die Umsetzung bevorzugt bei Temperaturen bis zu maximal 100°C, 90°C oder 80°C, besonders bevorzugt bis zu 70 C°, insbesondere bevorzugt bis zu 65 C°, am meisten bevorzugt bei Raumtemperatur durchgeführt.

Die Dauer der Umsetzung kann variieren und hängt insbesondere von den eingesetzten Thiophenen, der gewählten Temperatur und den verwendeten Oxidationsmitteln ab. Bevorzugt wird eine Umsetzung im Sekunden- oder Minutenbereich bis im Bereich weniger Stunden. Besonders bevorzugt ist die Umsetzung innerhalb von 10 Minuten bis 3 Stunden im Wesentlichen abgeschlossen, insbesondere bevorzugt innerhalb von 1,5 Stunden. Der Verlauf der Umsetzung kann durch dem Fachmann bekannte Maßnahmen analysiert werden.

Durch die Wahl geeigneter Reaktionsbedingungen lassen sich n-typ Halbleiter mittels chemisch oxidativer Umsetzung erhalten, die eine höhere Anzahl an Thiophen-Einheiten aufweisen. Ein weiterer Gegenstand der Erfindung sind daher auch n-typ Halbleiter der allgemeinen Formel (I), worin n eine ganze Zahl mit der Maßgabe 8<n≤40, bevorzugt 8<n≤32, ist.

Die durch die Umsetzung erhaltenen organischen n-typ Halbleiter können zur Entfernung von Rückständen und Nebenprodukten gewaschen werden. Bevorzugt sind mit den Lösungsmitteln der Umsetzung mischbare, gegebenenfalls wässrige, Waschlösungen.

Mit dem erfindungsgemäßen Verfahren zur Herstellung von n-typ Halbleitern steht ein effizientes Verfahren zur Verfügung. Bevorzugte n-typ Halbleiter sind daher nach dem oben beschriebenen Verfahren hergestellt.

Für die erfindungsgemäßen n-typ Halbleiter besteht ein breites Anwendungsspektrum auf dem Gebiet der elektrischen Einrichtungen, vor allem, wenn elektrisch isolierende Substrate mit elektrisch-leitfähigen Beschichtungen versehen werden sollen.

Ein weiterer Gegenstand ist daher die Verwendung der erfindungsgemäßen organischen n-typ Halbleiter zur Beschichtung von elektrisch isolierenden Substraten. Vorteilhaft ist, dass bereits bekannte Verfahren zum Aufbringen von Beschichtungen aus mittels oxidativer Polymerisierung hergestellten p-typ Halbleitern auf der Basis von Polythiophenen für die Beschichtung mit den erfindungsgemäßen organischen n-typ Halbleitern genutzt werden können. Als isolierende Substrate, die nicht oder nur schlecht leiten, kommen sowohl organische Materialien wie Polyethylene, Polypropylene, Polyvinylchlorid, Polyester, Polycarbonate und Polyamide, als auch anorganische wie Glas oder Keramik in Frage.

Aufgrund ihrer elastischen Eigenschaften eignen sich die erfindungsgemäßen organischen n-typ Halbleiter insbesondere zur Beschichtung von flexiblen Substraten, die elektrischleitfähig ausgestattet werden sollen. Vor allem flexible elektrisch isolierende Substrate aus organischen Kunststoffen, insbesondere Folien aus Polyethylenen, Polypropylenen, Polyvinylchlorid, Polyestern, Polycarbonaten und Polyamiden können mit den organischen n-typ Halbleitern ausgestattet werden. Bevorzugt ist daher die Verwendung der erfindungsgemäßen n-typ Halbleiter zur Beschichtung von flexiblen elektrisch isolierenden Substraten.

Aufgrund ihrer einfachen Handhabbarkeit hat sich der Einsatz von Dispersionen, die die organischen Halbleiter enthalten, für die Erzeugung von Beschichtungen als besonders vorteilhaft erwiesen. Dispersionen der organischen n-typ Halbleiter können erhalten werden, wenn diese in geeigneten Lösungsmitteln hergestellt und/oder in diese überführt werden. Es ist möglich, die Dispersionen in flüssiger Form weiterzuverarbeiten. Die Prozessierbarkeit in flüssiger Form eröffnet eine Vielzahl an Anwendungsmöglichkeiten. Dies schließt die Aufbringung der Dispersionen als feinste Tröpfchen auf ein Substrat durch drucktechnische Verfahren ein. Die Verwendung dieser Druckfluide soll jedoch nicht auf bestimmte Druckverfahren beschränkt verstanden werden. Beispielhaft sind Siebdruckverfahren, Tiefdruckverfahren, Flexodruckverfahren, Offsetdruckverfahren, Inkjetdruckverfahren oder Aerosoljetdruckverfahren als mögliche Anwendung genannt. Ein weiterer Gegenstand der Erfindung ist daher die Verwendung der erfindungsgemäßen n-typ Halbleiter in flüssigprozessierbaren Druckfluiden.

Flüssigprozessierbare Druckfluide mit einer Viskosität im Bereich von 10⁷ mPas bis 10¹ mPas eignen sich insbesondere für gängige Druckverfahren. Bevorzugt ist die Verwendung der erfindungsgemäßen n-Typ Halbleiter in flüssigprozessierbaren Druckfluiden mit einer Viskosität im Bereich von 10⁶ mPas bis 10² mPas.

Flüssigprozessierbare, scherverdünnende Druckfluide eignen sich für gängige Siebdruckverfahren. Ein weiterer Gegenstand der Erfindung ist daher die Verwendung der erfindungsgemäßen n-Typ Halbleiter in flüssigprozessierbaren, scherverdünnenden Druckfluiden.

Flüssigprozessierbare, scherverdünnende Druckfluide mit einer Viskosität im Bereich von 10⁷ mPas bei Scherraten von 10⁻³ 1/s und bei 10¹ mPas bei Scherraten von 10³ 1/s, eignen sich hervorragend für Siebdruckverfahren. Ein weiterer Gegenstand der Erfindung ist daher die Verwendung der erfindungsgemäßen n-Typ Halbleiter in flüssigprozessierbaren, scherverdünnenden Druckfluiden mit einer Viskosität im Bereich von 10⁷ mPas bei Scherraten von 10⁻³ 1/s und bei 10¹ mPas bei Scherraten von 10³ 1/s.

Bevorzugt ist die Verwendung der erfindungsgemäßen n-Typ Halbleiter in flüssigprozessierbaren, scherverdünnenden Druckfluiden mit einer Viskosität im Bereich von 10⁶ mPas bei Scherraten von 10⁻³ 1/s und bei 10² mPas bei Scherraten von 10³ 1/s.

Die kostengünstig hergestellten, organischen n-typ Halbleiter sind insbesondere für die Verwendung in thermoelektrischen Vorrichtungen wie thermoelektrischen Generatoren geeignet, in denen leitfähige Schichten aus n-typ- und p-typ Halbleitern zur Stromerzeugung genutzt werden. Beispielsweise in der DE 102012105086 B4 beschriebene organische thermoelektrische Generatoren mit Thermoelementen, in denen elektrisch isolierende organische Kunststofffolien mit organischen Halbleitern enthaltenden Beschichtungen elektrisch-leitend ausgerüstet sind, eröffnen neue Wege zu deren Herstellung und deren Einsatz. Ein weiterer Gegenstand der Erfindung ist daher die Verwendung der erfindungsgemäßen n-typ Halbleiter in thermoelektrischen Generatoren, insbesondere in organischen thermoelektrischen Generatoren.

Weitere Einzelheiten der vorliegenden Erfindung werden nachfolgend beispielhaft beschrieben, wobei die Erfindung jedoch in keiner Weise beschränkt werden soll.

### I. allgemeine schematische Darstellung der Herstellung eines n-typ Halbleiters ausgehend von 3,4-Ethylendioxythiophen (EDOT):

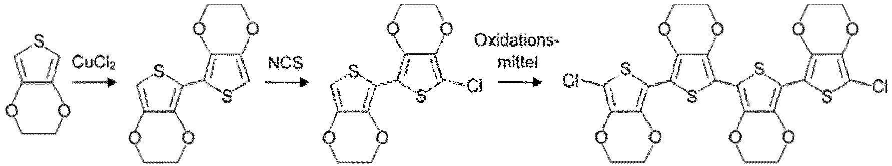

Der n-typ Halbleiter lässt sich über drei sehr einfache Reaktionsstufen darstellen. Mit EDOT, welches kommerziell für die PEDOT(Poly(3,4-Ethylendioxythiophen))-Synthese benutzt wird, steht ein sehr kostengünstig im Tonnenmaßstab erhältliches Ausgangsmaterial zur Verfügung. Eine als erster Schritt mögliche Ullmann-Kupplung funktioniert mit sehr guten Ausbeuten von bis zu 75%. Die darauf folgende Chlorierung lässt sich simpel mit N-Chlorsuccinimid (NCS) durchführen. Die anschließende, erfindungsgemäße chemisch oxidative Umsetzung des Dimers (BiEDOT) kann mittels eines preiswerten Eisen(III)salzes, beispielsweise Eisen(III)-Chlorid oder Eisen(III)-Tosylat, erfolgen.

### II. erfindungsgemäße Herstellung von n-typ Halbleitern in einem flüssigprozessierbaren Druckfluid:

### II.1 a) Chemisch oxidative Umsetzung von 7-chlor-2,2',3,3'-tetrahydro-5,5'-bithieno[3,4-b][1,4]dioxin (BiEDOT-Cl) :

### Beispiel 1:

### Lösung A

5 ml Tetraethylenglykol wurden zu 536 mg Fe-(III)-tosylat x 6 H₂O (852 µmol) zugegeben und die Mischung auf 200 °C erhitzt bis sich eine klare orangerote Lösung bildete. Die Lösung wurde anschließend auf Raumtemperatur abgekühlt.

### Lösung B

5 ml Tetraethylenglykol wurden zu 120 mg BiEDOT-Cl (379 µmol) zugegeben und die Mischung auf 200 °C erhitzt bis sich eine klare grüne Lösung bildete. Die Lösung wurde anschließend auf Raumtemperatur abgekühlt.

Zu der Lösung A wurde bei Raumtemperatur unter Rühren die Lösung B zugegeben, wobei sofort ein tief violett gefärbtes, Fluid erhalten wurde. Das Fluid wurde für weitere 120 Minuten gerührt.

### Beispiel 2:

### Lösung C

7,2 ml Tetraethylenglykol wurden zu 669 mg Fe-(III)-tosylat x 6 H₂O (1,06 mmol) zugegeben und die Mischung auf 200 °C erhitzt bis sich eine klare orangerote Lösung bildete. Die Lösung wurde anschließend auf 50 °C abgekühlt.

### Lösung D

10 ml Tetraethylenglykol wurden zu 120 mg BiEDOT-Cl (379 µmol) zugegeben und die Mischung auf 200 °C erhitzt bis sich eine klare grüne Lösung bildete. Die Lösung wurde anschließend auf 50 °C abgekühlt.

Zu der Lösung C wurde bei 50 °C unter Rühren die Lösung D zugegeben, wobei sofort ein dunkel violett gefärbtes, hochviskoses Fluid erhalten wurde. Das Fluid wurde für weitere 60 Minuten gerührt.

### II.1 b) Entfernung von Rückständen der oxidativen Umsetzung (Waschschritt):

Es wurden 10 ml eines 1:1 Ethylenglykol/Wasser-Gemischs als Extraktionsmittel zu dem Fluid gegeben und der Ansatz bei 1300 rpm für eine Stunde geschüttelt. Die Abtrennung des Extraktionsmittels wurde durch Dekantieren des Fluids nach einem Zentrifugationsschritt (10 Minuten bei 4200 rpm) erzielt. Der Extraktionsschritt wurde noch viermal wiederholt, wobei jeweils für 5 Minuten bei 4200 rpm zentrifugiert wurde.

### II.1 c) Bestimmung der Viskosität in Abhängigkeit der Scherrate (Schergeschwindigkeit):

### Messapparatur

Die Bestimmung der Viskosität wurde mit einem Anton Paar Physica MCR 101 Platte-Kegel Rheometer durchgeführt. Als Kegel wurde ein Kegel mit der Modellnummer PP25 verwendet, welcher durch dessen kleinere Geometrie für hochviskose Fluide geeignet ist. Die Temperatur während den Messungen betrug 25 °C.

### Vorgehen der Messung

Zur Messung wurden ca. 1 Gramm des Fluids auf den Teller des Rheometers aufgebracht und anschließend der Kegel durch die Apparatur automatisch abgesenkt. Während der Messung wurde der Kegel mit verschiedenen Schwerraten gedreht und die Viskosität über die Messapparatur aufgenommen. Der Messebereich der Scherrate wurde über die Software gesteuert und anschließend in Abhängigkeit zur ermittelten Viskosität grafisch aufgetragen.

### Rheologische Eigenschaften

Die Viskosität eines gemäß den Schritten a) und b) hergestellten Fluids lag im Bereich von 10⁶ mPas bei Scherraten von 10⁻³ 1/s und 10² mPas bei Scherraten von 10² 1/s. Hierbei nimmt die Anfangsviskosität bei steigenden Scherraten ab. Dieses Verhalten entspricht einem scherverdünnenden Fluid.

### II.1 d) Ermittlung der Leitfähigkeit und des Seebeck-Koeffizienten:

Das gewaschene Fluid wurden mittels Rakelschablone auf Glasobjektträger aufgetragen und bei ca. 220 °C mit Heißluft getrocknet.

Eine Trocknung des Fluids bei hiervon abweichenden Temperaturen, beispielsweise bei Temperaturen zwischen 150 °C und 220 °C, lieferte identische Ergebnisse. Alternativ zur Heißluft wurden auch Heizplatten zur Trocknung eingesetzt.

Silber-Elektroden wurden aufgebracht und die Leitfähigkeit sowie der Seebeck-Koeffizient der trockenen Beschichtung mit Hilfe eines Multimeters simultan bestimmt. Die Schichtdicke der Beschichtung wurde über ein DektakXT Profilometer bestimmt.

Der Seebeck-Koeffizient betrug -7,2 µV/K bei einer Leitfähigkeit der Beschichtung von 11 S/cm.

Eine gemäß den Schritten a) und b) hergestellte Dispersion der organischen n-typ Halbleiter erwies sich als stabil gegenüber Luftsauerstoff und zeigte über einen Zeitraum von mehr als 12 Monaten noch die Eigenschaften, die einem n-dotierten Material entsprechen.
11.2 Die Schritte II.1. a) bis c) wurden mit 7-brom-2,2',3,3'-tetrahydro-5,5'-bithieno[3,4-b][1,4]dioxin (BiEDOT-Br) durchgeführt. Der Seebeck-Koeffizient betrug -7,2 µV/K bei einer Leitfähigkeit der Beschichtung von 7 S/cm.
11.3 Die Schritte II.1. a) bis c) wurden mit 7-(2,2,3,3,3-pentafluorpropyl)-2,2',3,3'-tetrahydro-5,5'-bithieno[3,4-b][1,4]dioxin (BiEDOT-CH₂-CF₂-CF₃) durchgeführt. Der Seebeck-Koeffizient betrug -13,5 µV/K bei einer Leitfähigkeit der Beschichtung von 2 S/cm.

### III. Synthese von zur oxidativen Umsetzung eingesetzten Verbindungen:

Vorstufen für die Einführung von Substituenten mit negativem induktiven Effekt wurden nach bekannten Verfahren, beispielsweise mittels Ullmann-Kupplung wie in G. A. Sotzing, J. R. Reynolds, P.J. Steel in Advanced Materials 1997, 9, Seiten 795-798 oder G. A. Sotzing, J. R. Reynolds in Advanced Materials 1996, 8, Seiten 882-889 beschrieben, synthetisiert.

Zur Einführung eines Substituenten mit negativem induktiven Effekt werden beispielhaft die folgenden Substitutionen von BiEDOT beschrieben:

### III.1 Herstellung von 7-chlor-2,2',3,3'-tetrahydro-5,5'-bithieno[3,4-b][1,4]dioxin (BiEDOT-Cl):

Unter Stickstoffatmosphäre wurden 1.129 g (4 mmol) Bi(3,4-Ethylendioxythiophen) in 40 ml Dimethylformamid (DMF, dehyd.) gelöst. Zu dieser Lösung wurden tropfenweise 20 ml einer Lösung von 587 mg (4 mmol) N-Chlorsuccinimid in DMF zugegeben und die Temperatur des Reaktionsansatzes in einem Eisbad auf bis zu 10 °C gehalten. Anschließend wurde die Reaktionslösung für 2 Stunden in einem Ölbad auf eine Temperatur von 50 bis 60 °C erhitzt. Zur Fällung des Reaktionsprodukts wurden tropfenweise 100 ml Eiswasser zugegeben. Das Präzipitat wurde unter Vakuum abfiltriert und mit Methanol gewaschen. Es wurden 1.155 g dunkelgrünes Puder (Rohausbeute: 91%) erhalten, das gemäß dünnschichtchromatographischer Analyse und NMR-Spektren eine Mischung von monochloriertem und dichloriertem BiEDOT in einem Verhältnis von 1:6 war. Für die oxidative Umsetzung gemäß II. wurde auf eine Fraktionierung des Gemischs verzichtet. Gewünschtenfalls lässt sich jedoch eine Trennung nach bekannten Verfahren der Chromatographie, der fraktionierten Destillation, Kristallisation oder Extraktion vornehmen.
¹H-NMR(CDCl₃)-Spektrum: 4.22-4.24 (m, 2H), 4.29-4.33 (m, 6H), 6.27 (s, 1H).

### III.2 Herstellung von 7-brom-2,2',3,3'-tetrahydro-5,5'-bithieno[3,4-b][1,4]dioxin (BiEDOT-Br):

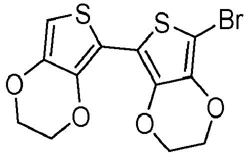

Bis-EDOT (1 g, 3,54 mmol, 1 eq.) wurde in 15 mL DMF gelöst und unter Rühren auf -15 °C gekühlt. Zu dieser Lösung wurde eine Lösung von N-Bromsuccinimid (630 mg, 3,54 mmol, 1 eq) in 10 mL DMF langsam zugetropft. Nach 1 h Rühren bei -15 °C wurde die Reaktionsmischung auf Raumtemperatur erwärmt und weitere 60 Minuten gerührt. Das Reaktionsgemisch wurde schließlich auf 150 g Eiswasser gegeben, mit Dichlormethan extrahiert (3 x 50 mL), und die organische Phase mit Natriumsulfat getrocknet und unter vermindertem Druck vom Lösungsmittel befreit. Es wurden 935 mg (Ausbeute: 73 %) eines bläulich-gelben Feststoffes erhalten.

### III.3 Herstellung von 7-(2,2,3,3,3-pentafluorpropyl)-2,2',3,3'-tetrahydro-5,5'-bithieno[3,4-b][1,4]dioxin (BiEDOT-CH₂-CF₂-CF₃):

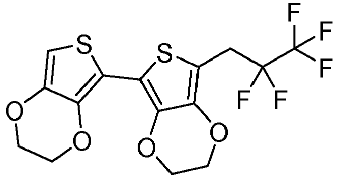

Bis-EDOT (1 g, 3,54 mmol, 1 eq.)wurde unter Argonatmosphäre in 35 mL absolutem THF gelöst und unter Rühren auf -78 °C gekühlt. Zu dieser Lösung wurde langsam n-BuLi (3,89 mmol, 1,1 eq. 1,56 mL einer 2,5 M Lösung in Hexan) zugetropft und es wurde 2 h bei -78 °C gerührt. Anschließend wurde langsam 1,1,1,2,2-pentafluoro-3-iodopropan (6 mmol, 1,56 g) zugetropft und weitere 50 Minuten bei -78 °C gerührt. Die Reaktionsmischung wurde schließlich auf RT erwärmt und mit Wasser und Dichlormethan verdünnt (jeweils 150 mL). Die organische Phase wurde abgetrennt, mit Natriumsulfat getrocknet, und unter vermindertem Druck vom Lösungsmittel befreit. Es wurden 1,35 g (Ausbeute: 92 %) eines gelben Feststoffes erhalten.

Sämtliche Merkmale der Erfindung können sowohl einzeln als auch in beliebiger Kombination miteinander erfindungswesentlich sein.

## Patentansprüche

1. Organische n-typ Halbleiter der allgemeinen Formel (I), 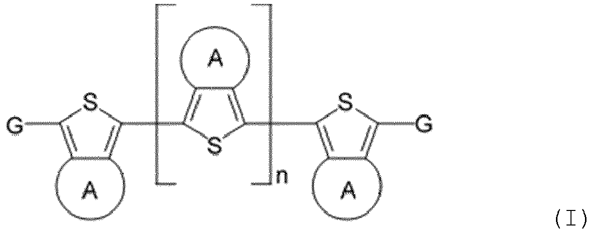 worin
A für einen gegebenenfalls ein oder mehrfach substituierten 5- oder 6-gliedrigen ungesättigten oder gesättigten heterocyclischen Ring mit bis zu zwei Heteroatomen steht, wobei die Heteroatome gleich oder verschieden sein können und ausgewählt sind aus der Gruppe aus Sauerstoffatomen und Schwefelatomen,
G ein Substituent mit einem negativen induktiven Effekt bedeutet,
und n für eine ganze Zahl von 2 bis 6 steht.

2. Organische n-typ Halbleiter nach Anspruch 1, **dadurch gekennzeichnet, dass** n für eine ganze Zahl von 2 bis 4 steht.

3. Organische n-typ Halbleiter nach Anspruch 1 oder 2, **dadurch gekennzeichnet, dass** G ausgewählt ist aus -O-Alkylgruppe, -OH, -NH-Alkylgruppe, -N-(Alkylgruppe)₂,-NH₂, -NH(CO)-Alkylgruppe, -O(CO)-Alkylgruppe, -O(CO)-Arylgruppe, -O(CO)-Phenylgruppe, Halogenalkylgruppe oder Halogenatom, bevorzugt ausgewählt ist aus Fluor, Chlor, Brom oder -CH₂-CF₂-CF₃.

4. Organische n-typ Halbleiter nach einem der Ansprüche 1 bis 3, **dadurch gekennzeichnet, dass** es solche der allgemeinen Formel (I-1) 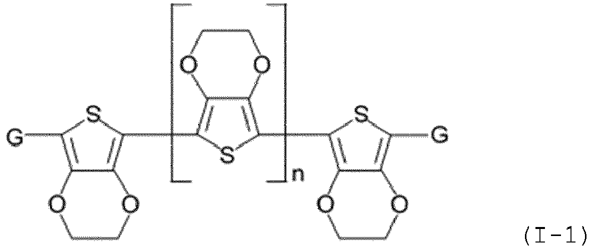 sind, worin G und n die in mindestens einem der Ansprüche 1 bis 3 angegebene Bedeutung haben.

5. Organische n-typ Halbleiter nach Anspruch 4, **dadurch gekennzeichnet, dass** es solche der allgemeinen Formel (I-1) sind, worin G Halogenalkylgruppe oder Halogenatom, bevorzugt Fluor, Chlor, Brom oder -CH₂-CF₂-CF₃, bedeutet.

6. Verfahren zur Herstellung von organischen n-typ Halbleitern der allgemeinen Formel (I), 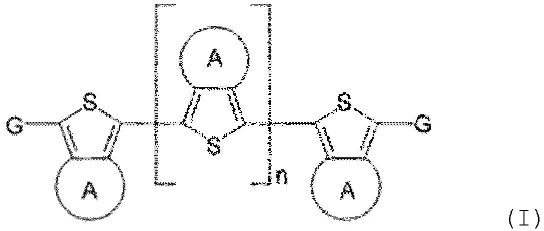 worin A, G und n die in mindestens einem der Ansprüche 1 bis 3 genannte Bedeutung haben,
durch chemisch oxidative Umsetzung von Thiophenen der allgemeinen Formel (II), 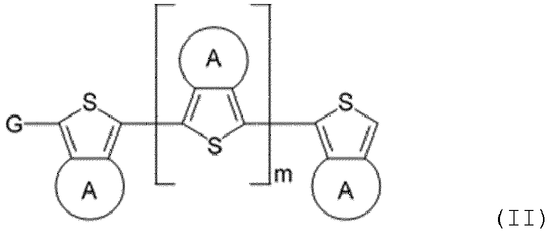 worin A und G die in der Formel (I) genannte Bedeutung haben, und m für 0, 1 oder 2 steht.

7. Verfahren nach Anspruch 6, **dadurch gekennzeichnet, dass** organische n-typ Halbleiter der Formel (I-1), worin G ein Chloratom bedeutet und n für eine ganze Zahl zwischen 2 und 4 steht, durch chemisch oxidative Umsetzung von Thiophenen der allgemeinen Formel (II-1), 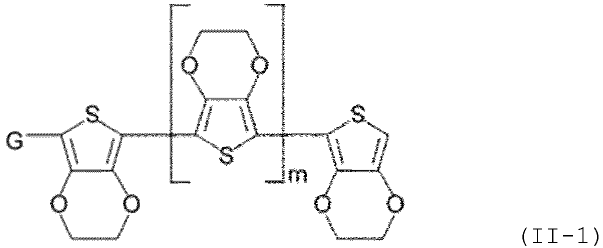 worin G ein Chloratom bedeutet und m für 0 oder 1 steht, erhalten werden.

8. Verfahren nach Anspruch 6 oder 7, **dadurch gekennzeichnet, dass** die Thiophene mit Eisen-(III)-Verbindungen chemisch oxidativ umgesetzt werden.

9. Verfahren nach einem der Ansprüche 6 bis 8, **dadurch gekennzeichnet, dass** die chemisch oxidative Umsetzung bei Temperaturen von -30°C bis 150°C vorgenommen wird.

10. Verfahren nach einem der Ansprüche 6 bis 9, **dadurch gekennzeichnet, dass** die chemisch oxidative Umsetzung für eine Dauer von 10 Minuten bis 3 Stunden vorgenommen wird.

11. Verwendung eines organischen n-typ Halbleiters nach einem der Ansprüche 1 bis 5 zur Beschichtung von elektrisch isolierenden Substraten.

12. Verwendung eines organischen n-typ Halbleiters nach einem der Ansprüche 1 bis 5 in flüssigprozessierbaren Druckfluiden.

13. Verwendung eines organischen n-typ Halbleiters nach einem der Ansprüche 1 bis 5 in flüssigprozessierbaren, scherverdünnenden Druckfluiden.

14. Verwendung eines organischen n-typ Halbleiters nach einem der Ansprüche 1 bis 5 in flüssigprozessierbaren, scherverdünnenden Druckfluiden mit einer Viskosität im Bereich von 10⁷ mPas bei Scherraten von 10⁻³ 1/s und bei 10¹ mPas bei Scherraten von 10³ 1/s.

15. Verwendung eines organischen n-typ Halbleiters nach einem der Ansprüche 1 bis 5 in thermoelektrischen Generatoren, bevorzugt in organischen thermoelektrischen Generatoren.
